# EUROPEAN PATENT SPECIFICATION

(11) **EP 1 285 917 B1**
(45) Date of publication and mention of the grant of the patent: **29.12.2004**
(21) Application number: 01926119.7
(22) Date of filing: 02.05.2001
(51) Int. Cl.: C07D 401/12

(54) **PROCESS FOR PRODUCING CARBOSTYRIL DERIVATIVE**
VERFAHREN ZUR HERSTELLUNG VON CARBOSTYRIL-DERIVATEN
PROCEDE DE PRODUCTION D'UN DERIVE CARBOSTYRILE

(43) Date of publication of application: 26.02.2003
(73) Proprietor: OTSUKA PHARMACEUTICAL CO., LTD., Chiyoda-ku Tokyo 101-8535 (JP)
(72) Inventor: AKI, Shinji, Itano-gun, Tokushima 779-0118 (JP); KURIMURA, Muneaki, Tokushima-shi, Tokushima 770-0002 (JP); NISHI, Takao, Itano-gun, Tokushima 771-0202 (JP); MINAMIKAWA, Jun-ichi, Naruto-shi, Tokushima 772-0011 (JP); TOMINAGA, Michiaki, Itano-gun, Tokushima 771-1346 (JP); FUKUYAMA, Norihiro, Tokushima-shi, Tokushima 770-8040 (JP); YAMAMOTO, Akihiro, Tokushima-shi, Tokushima 771-0130 (JP)
(74) Representative: HOFFMANN EITLE
(86) International application number: PCT/JP2001/003803
(87) International publication number: WO 2002/090351

(56) References cited:
- GB-A- 2 033 893
- JP-A- 1 265 051
- JP-A- 6 100 487
- JP-A- 11 152 243
- JP-A- 56 049 378
- NISHI T ET AL: "studies on 2-oxoquinoline derivatives as blood platelet aggregation inhibitors. I. alkyl 4-(2-oxo-1,2,3,4-tetrahydro-6-quinolyloxy) butyrates and related compounds" CHEMISTRY AND PHARMACEUTICAL BULLETIN, vol. 31, no. 3, 1983, pages 798-810, XP002235600
- FREEDMAN H.H. ET AL. TETRAHEDRON LETT. no. 38, 1975, pages 3251 - 3254, XP002942437

## Description

### TECHNICAL FIELD

The present invention relates to a novel process for producing carbostyril derivatives, and more particularly to a novel process for producing carbostyril derivatives represented by the following general formula (I): 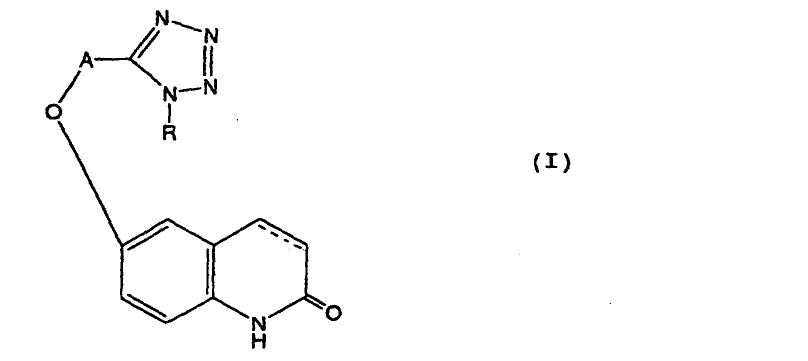 wherein A represents a group of the formula -(CH₂)₄-; R represents a cycloalkyl group; and the bond between the 3- and 4-positions of the carbostyril skeleton represents a single bond.

### BACKGROUND ART

The compound represented by the above-mentioned general formula (I), namely the objective compound of the present invention, is known to be useful as an antithrombotic agent, a cerebral circulation improver, an anti-inflammatory agent, an antiulcer agent, a hypotensive agent, an antiasthmatic agent, and a phosphodiesterase inhibitor, etc. (see: JP-A-56-49378 and USP No. 4,277,479).

Carbostyril derivatives represented by the general formula (I) have so far been produced by reacting a carbostyril derivative represented by the following general formula (II): 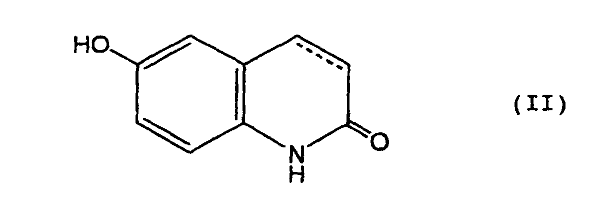 wherein the bond between the 3- and 4-positions of the carbostyril skeleton is a single or double bond, with a tetrazole derivative represented by the following general formula (III'): 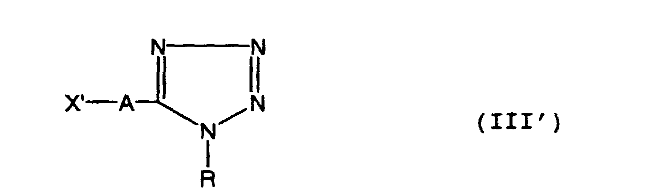 wherein X' represents a halogen atom, A represents a lower alkylene group and R represents a cycloalkyl group, in the presence of an inorganic base or an organic base (see: JP-A-56-49378; USP No. 4,277,479; and Chem. Pharm. Bull., 31(4), 1151-1157 (1983)).

### DISCLOSURE OF THE INVENTION

According to the above-mentioned known process, the yield of the compound of general formula (I) is as low as about 50 to 74%, because there is also formed a compound in which the tetrazole derivative of general formula (III') has reacted not only with the hydroxyl group of the carbostyril derivative of general formula (II) but also with the 1-position of the carbostyril derivative of general formula (II) simultaneously. Since the thus formed contaminative impurity is difficult to remove, production of a compound of general formula (I) having a high purity has required a complicated process of purification.

It is an object of the present invention to provide a process for producing a carbostyril derivative represented by the general formula (I) at a low cost and by a simple procedure. It is another object of the present invention to provide a process for producing a carbostyril derivative represented by the general formula (I) without any complicated process of purification, in a high yield, and in a high purity. It is yet another object of the present invention to provide an industrially advantageous process for producing the carbostyril derivatives represented by the general formula (I).

In view of the above-mentioned present situation, the present inventors have conducted various studies with the aim of achieving the above-mentioned objects. As a result, it has been found in the process of the studies surprisingly that, when a phase-transfer catalyst is used as a catalyst, a compound of general formula (I) given by a reaction between the hydroxyl group of the carbostyril derivative of general formula (II) and the tetrazole derivative of general formula (III') is formed, and a compound given by the reaction between the 1-position of the carbostyril derivative of general formula (I) and the tetrazole derivative of general formula (III') is scarcely formed, and the reaction progresses position-specifically, and thereby the objects of the present invention can be achieved. Based on this finding, the present invention has been accomplished.

According to the present invention, the objective carbostyril derivative represented by the general formula (I) can be obtained in a high yield and a high purity by reacting a carbostyril derivative represented by the following general formula (II): 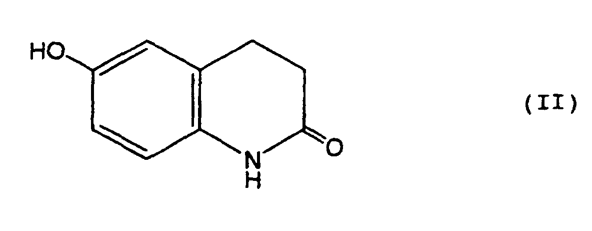 with a tetrazole derivative represented by the following general formula (III): 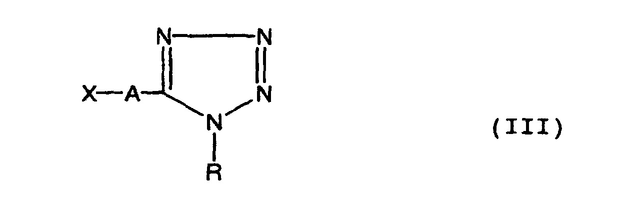 wherein X represents a halogen atom, A represents a tetramethylene group, and R represents a cyclohexyl group, in the presence of a phase-transfer catalyst in a solvent, which is water alone.

According to the process of the present invention, the hydroxyl group of the carbostyril derivative of general formula (II) and the tetrazole derivative of the general formula (III) can be made to react selectively and thereby the objective carbostyril derivative of general formula (I) can be produced on an industrial scale, at a low cost, by a simple procedure, in a high yield and in a high purity.

### BEST MODE FOR CARRYING OUT THE INVENTION

As the halogen atom represented by X in the general formula (III), mention can be made of fluorine atom, chlorine atom, bromine atom and iodine atom, among which particularly preferred is chlorine atom.

Next, the process of the present invention will be explained in more detail with reference to reaction schemes. 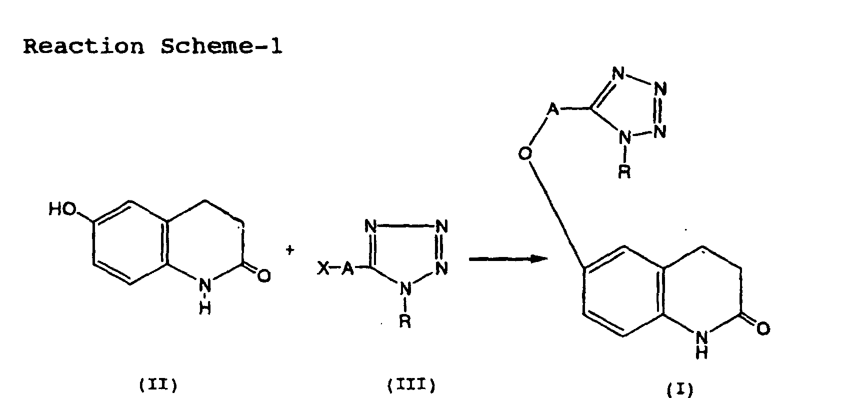 wherein X, A and R are as defined above.

In the reaction Scheme-1, the reaction between a compound of general formula (II) and a compound of general formula (III) is carried out in water alone as a solvent in the presence of a phase-transfer catalyst and further a basic compound.

As the basic compound, known ones can be used extensively. Examples thereof include inorganic bases such as sodium hydroxide, potassium hydroxide, cesium hydroxide, lithium hydroxide, sodium carbonate, potassium carbonate, cesium carbonate, lithium carbonate, sodium hydrogen carbonate, potassium hydrogen carbonate, silver carbonate and the like; alkali metals such as sodium, potassium and the like; alcoholates such as sodium methylate, sodium ethylate and the like; metallic salts of organic acids such as sodium acetate and the like; and organic bases such as triethylamine, diisopropylethylamine, pyridine, N,N-dimethylaniline, N-methylmorpholine, 4-dimethylaminopyridine, 1,5-diazabicyclo[4.3.0]non-5-ene (DBN), 1,8-diazabicyclo[5.4.0]undec-7-ene (DBU), 1,4-diazabicyclo[2.2.2]octane (DABCO) and the like. Among these bases, inorganic bases such as potassium carbonate, cesium carbonate, lithium carbonate and the like are particularly preferred.

As the phase transfer catalyst, mentioned can be made of, for example, quaternary ammonium salts substituted with a residue selected from the group consisting of straight or branched chain alkyl group having 1-18 carbon atoms, phenyl lower alkyl group and phenyl group, such as tetrabutylammonium chloride, tetrabutylammonium bromide, tetrabutylammonium fluoride, tetrabutylammonium iodide, tetrabutylammonium hydroxide, tetrabutylammonium hydrogen sulfate, tributylmethylammonium chloride, tributylbenzylammonium chloride, tetrapentylammonium chloride, tetrapentylammonium bromide, tetrahexylammonium chloride, benzyldimethyloctylammonium chloride, methyltrihexylammonium chloride, benzylmethyloctadecanylammonium chloride, methyltridecanylammonium chloride, benzyltripropylammonium chloride, benzyltriethylammonium chloride, phenyltriethylammonium chloride, tetraethylammonium chloride, tetramethylammonium chloride and the like; phosphonium salts substituted with a residue selected from the group consisting of straight or branched chain alkyl groups having 1-18 carbon atoms such as tetrabutylphosphonium chloride and the like; and pyridinium salts substituted with a straight or branched chain alkyl group having 1-18 carbon atoms such as 1-dodecanylpyridinium chloride and the like. Among these phase transfer catalysts, quaternary ammonium salts substituted with a straight or branched chain alkyl group having 1-18 carbon atoms such as tetrabutylammonium chloride and the like are particularly preferred. As the salt-forming ions in these salts, hydroxyl ion, hydrogen sulfate ion and halogen ions are preferred, among which chlorine ion is particularly preferred. If desired, sodium sulfite or the like may be added to the reaction system of the above-mentioned reaction for the purpose of preventing the coloration caused by oxidation.

The reaction is carried out usually at a temperature not lower than ambient temperature and not higher than 200°C, and preferably at a temperature of 50-150°C. The reaction time is usually from about one hour to about 10 hours. It is recommended to use the compound (III) usually in an amount of at least 0.5 mol and preferably 0.5-1.5 mol per mol of the compound (II), to use the basic compound usually in an amount of 1-5 mol per mol of the compound (II), and to use the phase transfer catalyst usually in an amount of 0.1-1 mol and preferably 0.1-0.5 mol per mol of the compound (II).

The compound of general formula (I) obtained by the above-mentioned reaction can easily be isolated by the conventional separating means. As said separating means, mention can be made of, for example, extraction method using a solvent, dilution method, recrystallization method, column chromatography, preparative thin layer chromatography, etc.

### Examples

Next, the process of the present invention is more concretely explained below with reference to examples. The invention is by no means limited thereby.

### Reference Example 1

Into a three-necked flask having a capacity of 300 ml were introduced 10.00 g of 6-hydroxy-3,4-dihydrocarbostyril, 16.36 g of 1-cyclohexyl-5-(4-chlorobutyl)-1,2,3,4-tetrazole, 10.16 g of potassium carbonate, 3.00 g of tetrabutylammonium chloride, 0.05 g of sodium sulfite, 30 ml of toluene and 50 ml of water. The content of the flask was heated under reflux for 8 hours. After cooling the reaction mixture to ambient temperature, the deposited crystalline product was collected by filtration and washed with 50 ml of water. Then, the crude crystal thus obtained was introduced into 70 ml of 90% methanol cooled to 5°C, and stirred at 5°C for 10 minutes for the sake of washing. The crystal was collected by filtration and further washed on the suction filter with 20 ml of 90% methanol cooled to 5°C. The crystal was dried to obtain 21.46 g (yield 95%) of 6-[4-(1-cyclohexyl-1,2,3,4-tetrazol-5-yl)butoxy]-3,4-dihydrocarbostyril as a colorless needle-like crystalline product. Purity: 99.80%; m.p.: 158-159°C

The purity was measured by high performance liquid chromatography under the following conditions:

| | |
|---|---|
| Column | YMC Pack SIL A-002 (manufactured by YMC Co.) |
| Moving phase | dichloromethane/n-hexane/methanol = 20/10/1 |
| Detector | UV, 254 nm |
| Flow rate | 0.90 ml/min. |
| Retention time | 4.7 min. |

### Example 2

Into a flask having a capacity of 200 ml were introduced 12.00 g of 6-hydroxy-3,4-dihydrocarbostyril, 19.60 g of 1-cyclohexyl-5-(4-chlorobutyl)-1,2,3,4-tetrazole, 8.20 g of 50% aqueous solution of tetrabutylammonium chloride, 12.20 g of potassium carbonate, 0.60 g of sodium sulfite and 60 ml of water. The content of the flask was heated under reflux for 8 hours with stirring. After the reaction, the reaction mixture was cooled to ambient temperature, and the deposited crude crystal was once collected by filtration. After washing the crystal firstly with 36 ml of methanol and then with 60 ml of water, the crystal was again introduced into a flask having a capacity of 200 ml and heated under reflux together with 84 ml of methanol for 2 hours. The solution thus obtained was cooled to 10°C. The crystal was collected by filtration, washed firstly with 24 ml of methanol and then with 24 ml of water, and dried at 80°C. Thus, 23.84 g (yield 87.7%) of 6-[4-(1-cyclohexyl-1,2,3,4-tetrazol-5-yl)butoxy]-3,4-dihydrocarbostyril was obtained as a colorless needle-like crystalline product.
Purity: 99.89%; m.p.: 158-159°C

The purity was measured by high performance liquid chromatography (HPLC) under the same conditions as in Reference Example 1.

## Claims

1. A process for producing a carbostyril derivative represented by the following general formula (I): 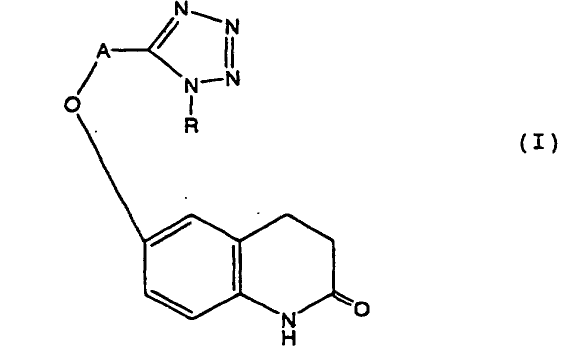 wherein A represents a group of the formula: (CH₂)₄-, and R represents a cyclohexyl group, which comprises reacting a carbostyril derivative represented by the following general formula (II): 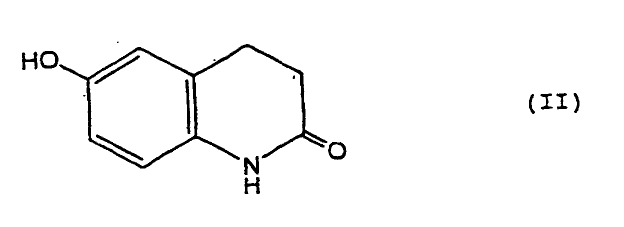 with a tetrazole derivative represented by the following general formula (III) : 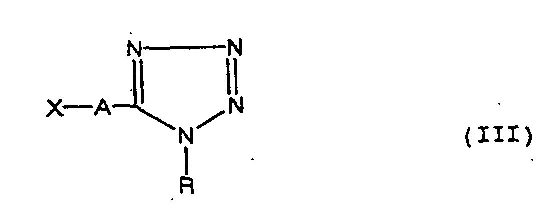 wherein X represents a halogen atom, and A and R are as defined above, in the presence of a phase transfer catalyst in a solvent which is water alone.

2. A process for producing a carbostyril derivative according to Claim 1, wherein the reaction is carried out at a reaction temperature not lower than the ambient temperature and not higher than 200°C in the presence of a basic compound.

3. A process for producing a carbostyril derivative according to Claim 2, wherein the reaction temperature is 50°C to 150°C.

4. A process for producing a carbostyril derivative according to Claim 2, wherein said basic compound is an inorganic base.

5. A process for producing a carbostyril derivative according to Claim 1, wherein X in the tetrazole derivative represented by general formula (III) is a chlorine atom.

6. A process for producing a carbostyril derivative according to Claim 1, wherein said phase transfer catalyst is a quaternary ammonium salt substituted with a residue selected from the group consisting of straight or branched chain alkyl groups having 1-18 carbon atoms, phenyl lower alkyl groups and phenyl groups, a phosphonium salt substituted with a straight or branched chain alkyl group having 1-18 carbon atoms, or a pyridinium salt substituted with a straight or branched chain alkyl group having 1-18 carbon atoms, and the salt-forming ion in these salts is a hydroxyl ion, a hydrogen sulfate ion or a halogen ion.

7. A process for producing a carbostyril derivative according to Claim 6, wherein said phase transfer catalyst is a quaternary ammonium salt substituted with a residue selected from the group consisting of straight or branched chain alkyl groups having 1-18 carbon atoms, phenyl lower alkyl groups and phenyl groups, and the salt forming ion in this said salt is a halogen ion.

8. A process for producing a carbostyril derivative according to Claim 7, wherein said phase transfer catalyst is a quaternary ammonium salt substituted with a straight or branched chain alkyl group having 1-18 carbon atoms.

9. A process for producing a carbostyril derivative according to Claim 7, wherein said salt-forming ion in the salt is a chlorine ion.

10. A process for producing a carbostyril derivative according to Claim 7, wherein said phase transfer catalyst is tetrabutylammonium chloride.

11. A process for producing a carbostyril derivative according to Claim 10, wherein said phase transfer catalyst is used in an amount of 0.1 to 1 mol per mol of the compound of general formula (II).

12. A process for producing a carbostyril derivative according to Claim 11, wherein said phase transfer catalyst is used in an amount of 0.1 to 0.5 mol per mol of the compound of general formula (II).

13. A process for producing a carbostyril derivative according to Claim 1,
wherein the reaction is carried out in the presence of an agent for preventing the coloration caused by oxidation.

14. A process for producing a carbostyril derivative according to Claim 13, wherein the agent for preventing the coloration caused by oxidation is sodium sulfite.

15. A process for producing a carbosytril derivative according to Claim 4, wherein said inorganic base is chosen from potassium carbonate, cesium carbonate, cesium hydroxide, lithium carbonate, lithium hydroxide, sodium hydroxide, potassium hydroxide, potassium hydrogen carbonate, sodium carbonate, sodium hydrogen carbonate and mixtures thereof.

## Patentansprüche

1. Verfahren zur Herstellung eines Carbostyrilderivats, das durch die folgende allgemeine Formel (I) wiedergegeben wird: 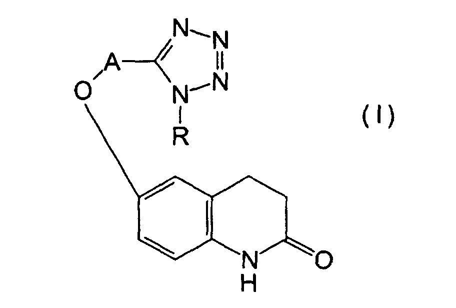 worin A eine Gruppe der Formel -(CH₂)₄- bedeutet und R eine Cyclohexylgruppe bedeutet, welches die Umsetzung eines Carbostyrilderivats, das durch die folgende allgemeine Formel (II) wiedergegeben ist: 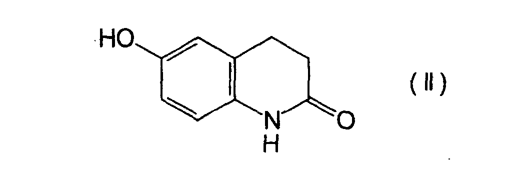 mit einem Tetrazolderivat, das durch die folgende allgemeine Formel (III) wiedergegeben wird: 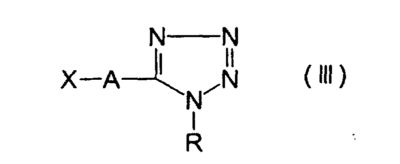 worin X ein Halogenatom bedeutet und A und R wie oben definiert sind, in Gegenwart eines Phasentransferkatalysators in einem Lösungsmittel, welches Wasser alleine ist, umfasst.

2. Verfahren zur Herstellung eines Carbostyrilderivats gemäss Anspruch 1, wobei die Reaktion bei einer Reaktionstemperatur von nicht niedriger als Umgebungstemperatur und nicht höher als 200°C in Gegenwart einer basischen Verbindung durchgeführt wird.

3. Verfahren zur Herstellung eines Carbostyrilderivats gemäss Anspruch 2, wobei die Reaktionstemperatur 50 bis 150°C ist.

4. Verfahren zur Herstellung eines Carbostyrilderivats gemäss Anspruch 2, wobei die basische Verbindung eine anorganische Base ist.

5. Verfahren zur Herstellung eines Carbostyrilderivats gemäss Anspruch 1, wobei X im Tetrazolderivat mit der allgemeinen Formel (III) ein Chloratom ist.

6. Verfahren zur Herstellung eines Carbostyrilderivats gemäss Anspruch 1, wobei der Phasentransferkatalysator ein quaternäres Ammoniumsalz, das substituiert ist mit einem Rest, ausgewählt aus der Gruppe bestehend aus gerad- oder verzweigtkettigen Alkylgruppen mit 1 bis 18 Kohlenstoffatomen, Phenyl-niederalkyl-Gruppen und Phenylgruppen, ein Phosphoniumsalz, das mit einer gerad- oder verzweigtkettigen Alkylgruppe mit 1 bis 18 Kohlenstoffatomen substituiert ist, oder ein Pyridiniumsalz ist, das mit einer gerad- oder verzweigtkettigen Alkylgruppe mit 1 bis 18 Kohlenstoffatomen substituiert ist, und das salzbildende Ion in diesen Salzen ein Hydroxylion, ein Hydrogensulfation oder ein Halogenion ist.

7. Verfahren zur Herstellung eines Carbostyrilderivats gemäss Anspruch 6, wobei der Phasentransferkatalysator ein quaternäres Ammoniumsalz ist, das substituiert ist mit einem Rest, ausgewählt aus der Gruppe bestehend aus gerad- oder verzweigtkettigen Alkylgruppen mit 1 bis 18 Kohlenstoffatomen, Phenyl-niederalkyl-Gruppen und Phenylgruppen, und das salzbildende Ion in diesem Salz ein Halogenion ist.

8. Verfahren zur Herstellung eines Carbostyrilderivats gemäss Anspruch 7, wobei der Phasentransferkatalysator ein quaternäres Ammoniumsalz ist, das mit einer gerad- oder verzweigtkettigen Alkylgruppe mit 1 bis 18 Kohlenstoffatomen substituiert ist.

9. Verfahren zur Herstellung eines Carbostyrilderivats gemäss Anspruch 7, wobei das salzbildende Ion in dem Salz ein Chlorion ist.

10. Verfahren zur Herstellung eines Carbostyrilderivats gemäss Anspruch 7, wobei der Phasentransferkatalysator Tetrabutylammoniumchlorid ist.

11. Verfahren zur Herstellung eines Carbostyrilderivats gemäss Anspruch 10, wobei der Phasentransferkatalysator in einer Menge von 0,1 bis 1 mol pro Mol der Verbindung der allgemeinen Formel (II) verwendet wird.

12. Verfahren zur Herstellung eines Carbostyrilderivats gemäss Anspruch 11, wobei der Phasentransferkatalysator in einer Menge von 0,1 bis 0,5 mol pro Mol der Verbindung der allgemeinen Formel (II) verwendet wird.

13. Verfahren zur Herstellung eines Carbostyrilderivats gemäss Anspruch 1, wobei die Reaktion in Gegenwart eines Mittels zur Verhinderung von Verfärbung, die durch Oxidation verursacht wird, durchgeführt wird.

14. Verfahren zur Herstellung eines Carbostyrilderivats gemäss Anspruch 13, wobei das Mittel zur Verhinderung der Verfärbung, die durch Oxidation verursacht wird, Natriumsulfit ist.

15. Verfahren zur Herstellung eines Carbostyrilderivats gemäss Anspruch 4, wobei die anorganische Base ausgewählt ist aus Kaliumcarbonat, Cäsiumcarbonat, Cäsiumhydroxid, Lithiumcarbonat, Lithiumhydroxid, Natriumhydroxid, Kaliumhydroxid, Kaliumhydrogencarbonat, Natriumcarbonat, Natriumhydrogencarbonat und Mischungen davon.

## Revendications

1. Procédé de production d'un dérivé carbostyrile représenté par la formule générale (I) suivante : 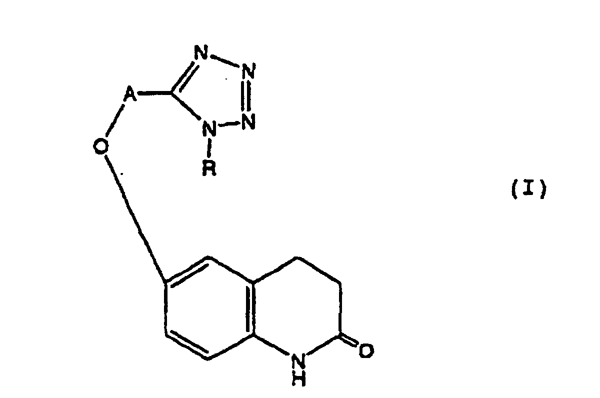 dans laquelle A représente un groupe de formule : -(CH₂)₄-, et R représente un groupe cyclohexyle, qui comprend la réaction d'un dérivé carbostyrile représenté par la formule générale (II) suivante : 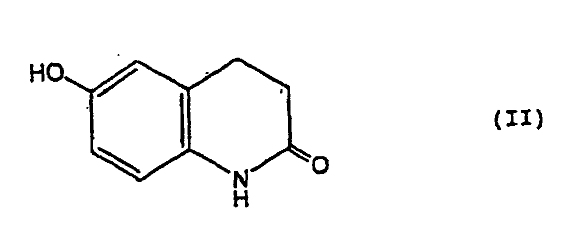 avec un dérivé tétrazole représenté par la formule générale (III) suivante : 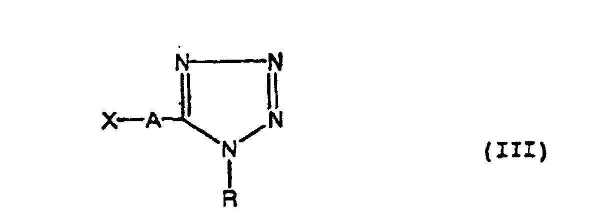 dans laquelle X représente un atome d'halogène, et A et R sont tels que définis ci-dessus, en présence d'un catalyseur de transfert de phase dans un solvant qui est l'eau seule.

2. Procédé de production d'un dérivé carbostyrile selon la revendication 1, dans lequel la réaction est réalisée à une température de réaction n'étant pas inférieure à la température ambiante et pas supérieure à 200°C en présence d'un composé basique.

3. Procédé de production d'un dérivé carbostyrile selon la revendication 2, dans lequel la température de réaction est de 50°C à 150°C.

4. Procédé de production d'un dérivé carbostyrile selon la revendication 2, dans lequel ledit composé basique est une base inorganique.

5. Procédé de production d'un dérivé carbostyrile selon la revendication 1, dans lequel X dans le dérivé tétrazole représenté par la formule générale (III) est un atome de chlore.

6. Procédé de production d'un dérivé carbostyrile selon la revendication 1, dans lequel ledit catalyseur de transfert de phase est un sel d'ammonium quaternaire substitué par un résidu choisi dans le groupe constitué par les groupes alkyles à chaîne linéaire ou ramifiée ayant de 1 à 18 atomes de carbone, les groupes phényl alkyles inférieurs et les groupes phényles, un sel de phosphonium substitué par un groupe alkyle à chaîne linéaire ou ramifiée ayant de 1 à 18 atomes de carbone, ou un sel de pyridinium substitué par un groupe alkyle à chaîne linéaire ou ramifiée ayant de 1 à 18 atomes de carbone, et l'ion formant un sel dans ces sels est un ion hydroxyle, un ion sulfate d'hydrogène ou un ion halogène.

7. Procédé de production d'un dérivé carbostyrile selon la revendication 6, dans lequel ledit catalyseur de transfert de phase est un sel d'ammonium quaternaire substitué par un résidu choisi dans le groupe constitué par les groupes alkyles à chaîne linéaire ou ramifiée ayant de 1 à 18 atomes de carbone, les groupes phényl alkyles inférieurs et les groupes phényles, et l'ion formant un sel dans ce dit sel est un ion halogène.

8. Procédé de production d'un dérivé carbostyrile selon la revendication 7, dans lequel ledit catalyseur de transfert de phase est un sel d'ammonium quaternaire substitué par un groupe alkyle à chaîne linéaire ou ramifiée ayant de 1 à 18 atomes de carbone.

9. Procédé de production d'un dérivé carbostyrile selon la revendication 7, dans lequel ledit ion formant un sel dans le sel est un ion chlore.

10. Procédé de production d'un dérivé carbostyrile selon la revendication 7, dans lequel ledit catalyseur de transfert de phase est le chlorure de tétrabutylammonium.

11. Procédé de production d'un dérivé carbostyrile selon la revendication 10, dans lequel ledit catalyseur de transfert de phase est utilisé en une quantité de 0,1 à 1 mole par mole du composé de formule générale (II).

12. Procédé de production d'un dérivé carbostyrile selon la revendication 11, dans lequel ledit catalyseur de transfert de phase est utilisé en une quantité de 0,1 à 0,5 mole par mole du composé de formule générale (II).

13. Procédé de production d'un dérivé carbostyrile selon la revendication 1, dans lequel la réaction est réalisée en présence d'un agent de prévention de la coloration provoquée par l'oxydation.

14. Procédé de production d'un dérivé carbostyrile selon la revendication 13, dans lequel l'agent de prévention de la coloration provoquée par l'oxydation est le sulfite de sodium.

15. Procédé de production d'un dérivé carbostyrile selon la revendication 4, dans lequel ladite base inorganique est choisie parmi le carbonate de potassium, le carbonate de césium, l'hydroxyde césium, le carbonate de lithium, l'hydroxyde de lithium, l'hydroxyde de sodium, l'hydroxyde de potassium, l'hydrogénocarbonate de potassium, le carbonate de sodium, l'hydrogénocarbonate de sodium et les mélanges de ceux-ci.
